# EUROPÄISCHE PATENTSCHRIFT

(11) **EP 0 919 547 B1**
(45) Veröffentlichungstag und Bekanntmachung des Hinweises auf die Patenterteilung: **05.06.2002**
(21) Anmeldenummer: 98121943.9
(22) Anmeldetag: 19.11.1998
(51) Int. Cl.: C07C 403/14, C07C 45/59, C07C 45/67, C07D 317/12, C07D 317/22, C07D 317/26

(54) **Herstellung von Polyenaldehyden**
Preparation of polyenaldehydes
Préparation d'aldéhydes polyéniques

(30) Priorität: 27.11.1997 EP 97120814
(43) Veröffentlichungstag der Anmeldung: 02.06.1999
(73) Patentinhaber: F. HOFFMANN-LA ROCHE AG, 4070 Basel (CH)
(72) Erfinder: Reuttimann, August, 4144 Arlesheim (CH)

(56) Entgegenhaltungen:
- EP-A- 0 268 460
- EP-A- 0 432 021
- EP-A- 0 816 334
- DE-B- 1 031 301
- FR-A- 2 338 241
- FR-A- 2 681 784
- US-A- 4 832 059
- CHEMICAL ABSTRACTS, vol. 57, no. 13, 24. Dezember 1962 Columbus, Ohio, US; abstract no. 16671c, ZH.A. KRASNAYA ET AL: "A new path for the synthesis of vitamin A from beta-ionylideneacetaldehyde" Seite 16671; Spalte 1; XP002041881 & ZH. OBSHCH. KHIM., Bd. 32, 1962, Seiten 64-70,
- CHEMICAL ABSTRACTS, vol. 52, no. 14, 25. Juli 1958 Columbus, Ohio, US; abstract no. 11737e, I.N. NAZAROV ET AL: "Condensation of acetals with ethoxyisopropene. New method of synthesis of polyene aldehydes of isoprenoid type" Seite 11737; Spalte 1; XP002041882 & DOKLADY AKAD. NAUK S.S.S.R., Bd. 118, 1958, Seiten 716-719,
- DATABASE CHEMLIST CHEMICAL ABSTRACTS SERVICE (ACS) XP002095179 & ANNEX TO OFFICIAL JOURNAL OF THE EUROPEAN COMMUNITIES,15. Juni 1990,

## Beschreibung

Die vorliegende Erfindung betrifft ein Verfahren zur Herstellung von Polyenaldehyden aus acetalisierten Polyenaldehyden kürzerer Kettenlänge durch eine säurekatalysierte Kondensationsreaktion mit Alkoxydienen.

Lewissäure-katalysierte Additionen von α,β-ungesättigten Ethern (Enolethern) an Acetale sind schon lange bekannt und gehen auf die Arbeiten von Müller-Cunradi und Pieroh zurück (siehe US-Patentschrift 2.165.962). Hoaglin und Hirsch [J.A.C.S. 71, 3468 ff. (1949)] haben diese Reaktion weiter untersucht und die Anwendungsmöglichkeiten erweitert, was ebenfalls Isler et al. in den fünfziger Jahren getan haben, und zwar auf die Synthese von β-Carotin, Crocetindialdehyd, Lycopin sowie β-Apocarotinoiden [siehe Helv. Chim. Acta 39, 249 ff. und 463 ff. (1956), ibid. 42, 854 ff. (1959) sowie US-Patentschriften 2.827.481 und 2.827.482]. Später erweiterte Mukaiyama die Reaktion durch Verwendung der gut und leicht zugänglichen Trimethylsilylenolether [Angew. Chem. 89, 858 ff. (1977) und Org. Reactions 28, 203 ff. (1982)].

Reaktionen von Ethenolalkylethern mit cyclischen Acetalen, z.B. Ethylenacetalen, sind ebenfalls bekannt; sie ergeben durch Addition zwei Ringsauerstoffatome aufweisende cyclische Verbindungen, z.B. 1,4-Dioxacycloheptane [siehe Mikhailov et al., Izv. Akad. Nauk. SSSR, Otd. Khim. Nauk. 1960, 1903 ff./Chem. Abs. 55, 13409 f(1961), und Deutsche Patentschrift 1.031.301/Chem. Abs. 54, 22712 d (1960)].

Ueber die ersten Lewissäure-katalysierten Kondensationen von 1-Alkoxy-1,3-dienen (Dienolethern) mit α,β-ungesättigten Acetalen wurde von Nazarov und Krasnaya [J. Gen. Chem. USSR 28, 2477 ff. (1958) und Dokl. Akad. Nauk S.S.S.R. 118, 716 - 719 (1958) / Chem. Abs. 52, No. 14, 11737e (1958)] und von Makin [Pure & Appl. Chem. 47, 173 ff. (1976), J. Gen. Chem. USSR 31, 3096 ff. (1961) und 32, 3112 ff. (1962)] berichtet. Dabei erfolgt die Kopplung des Acetals an den Dienolether soweit ersichtlich ausschliesslich an dessen γ-Stellung unter Bildung eines kettenverlängerten α,β-ungesättigten Acetals, das jedoch in Konkurrenz zum ersten Acetal mit weiterem Dienolether reagiert unter Bildung eines weiteren, kettenverlängerten α,β-ungesättigten Acetals usw. [Telomerbildung; siehe auch noch Chemla et al., Bull. Soc. Chim. Fr. 130, 200 ff. (1993)]. Aus diesem Grund ist eine derartige Kondensation für synthetische Zwecke, speziell für die Synthese von Apocarotinoiden, als nicht brauchbar befunden worden [Isler et al., Adv. Org. Chem. 4, 115 ff. (1963)].

Zh. Obshch. Khim. 32 64-70 (1962) / Chem. Abs. 57, No. 13, 16671c (1962) beschreibt gemäss dem obigen Prinzip die Kondensation von β-Jonolidenacetaldehyd-Diethylacetal mit 1-Ethoxy-3-methyl-1,3-butadien in Gegenwart von Zinkchlorid zum Etheracetal in 62%iger Ausbeute, die anschliessende quantitative Hydrolyse des Etheracetals mit Orthophosphorsäure zum δ-Etheraldehyd von Vitamin A, und schliesslich die Abspaltung des Alkohols vom δ-Etheraldehyd durch Chromatographie an Alumina, um Vitamin A-Aldehyd herzustellen. In diesem Artikel ist allerdings die Telomerbildung nicht erwähnt.

Nicht nur 1-Alkoxy-1,3-diene, sondern auch Trimethylsilyloxydiene [des Typs CH₂=CH-CH=CH-OSi(CH₃)₃] können in Gegenwart von Lewissäure-Katalysatoren mit α,β-ungesättigten Acetalen kondensiert werden, wie dies Mukaiyama et al. in Chem. Lett. 1975, 319 ff. offenbarten. Auch bei dieser Kopplung scheint der Angriff ausschliesslich am endständigen (γ-) Kohlenstoffatom des Diensystems zu erfolgen, um "γ-Produkte" zu bilden [siehe Mukaiyama et al., Bull. Chem. Soc. Japan 50, 1161 ff. (1977) sowie Japanische Patentpublikation (Kokai) 36.645/1977/Chem. Abs. 87, 201825 t (1977)]. Im Gegensatz zur Reaktion mit 1-Alkoxy-1,3-dienen, bei welcher ein α,β-ungesättigtes Acetal anfällt, wird bei der Umsetzung von Trimethylsilyloxydienen mit Acetalen ein Aldehyd gebildet, der mit dem Dien nicht weiterreagieren kann (keine Telomerbildung). Dabei werden als Katalysatoren Zinkbromid und viele andere Lewissäuren nur in kleinen Mengen benötigt [Fleming (et al.), Tetr. Lett. 1979, 3209 ff. und Chimia 34, 265 ff. (1980) sowie Brownbridge, Synth. 1983, 85 ff.]. Unter Verwendung dieser Methode konnten Mukaiyama et al. Vitamin A synthetisieren [siehe Kokai 36.645/1977, Chem. Lett. 1975, 1201 ff. sowie Bull. Chem. Soc. Japan 51, 2077 ff. (1978)] und Mitarbeiter von Rhône-Poulenc neue Zugänge zu Carotinoiden und Vitamin A entwickeln (siehe DOS 2.701.489 und A.E.C. Société de Chimie Organique et Biologique Nr. 7824350).

Die obenerwähnte, auf den Arbeiten von Nazarov und Krasnaya, Makin sowie Chemla et al. basierende Lewissäure-katalysierte Kondensation eines Dienolethers mit einem α,β-ungesättigten Acetal würde einen sehr wertvollen Zugang zu Apocarotinalen und Bis-apocarotinalen eröffnen, falls die Ausbeute am gewünschten Primärprodukt des Typs ...CH=CH-CH(OAlkyl¹)-CH₂-CH=CH-CH(OAlkyl¹)(OAlkyl²) erhöht bzw. die Telomerbildung unterdrückt werden könnte. So könnte aus diesem Primärprodukt durch Hydrolyse der Acetalgruppe C(OAlkyl¹)(OAlkyl²) und Elimination von Alkyl¹OH der gewünschte Polyenaldehyd des Typs CH=CH-CH=CH-CH=CH-CHO erhalten werden. Zusätzlich zu der Situation, dass bei dieser Reaktion die Bildung der Doppelbindung unter katalytischen Bedingungen erfolgt, werden keine phosphor-, silizium- bzw. schwefelhaltigen Reagenzien benötigt.

Weniger bekannt aus der Fachliteratur ist die Kopplung eines α,β-ungesättigten Ethylenacetals mit einem Trimethylsilyloxydien. Es entsteht dabei das zu den "üblichen" Dienoletherkondensationen mit Dialkylacetalen analoge Produkt in mässiger Ausbeute [siehe nochmals Chem. Lett. 1975, 319 ff. sowie Bull. Chem. Soc. Japan 50, 1161 ff. (1977)], und zwar gemäss der Gleichung:

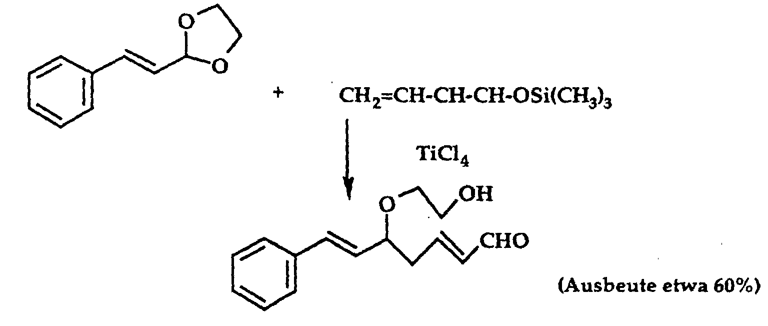

Die am 7. Januar 1998 veröffentlichte europäische Patentpublikation EP 0816334 A1 beschreibt ein Verfahren zur Herstellung von Polyenaldehyden und-dialdehyden durch Umsetzung eines Polyen-O,O-dialkylacetals bzw. -di(O,O-dialkylacetals) mit einem Dienolether in Gegenwart einer Lewis- oder Brönstedsäure, anschliessende Hydrolyse des Reaktionsgemisches und schliesslich Abspaltung des Alkohols von dem durch die Hydrolyse erzeugten Polyenderivat unter basischen Bedingungen.

Ziel der vorliegenden Erfindung ist es, ausgehend von Polyenacetalen kettenverlängerte Polyenaldehyde herzustellen, und zwar unter möglichst weitgehender Vermeidung der obenerwähnten Nachteile des Standes der Technik sowie mit Ersatz der bisher zu diesem Zweck verwendeten Wittig-, Horner- oder Julia-Reaktion. Dieses Ziel wird erfindungsgemäss erreicht, indem man ein Polyen-O,O-ethylenacetal mit einem 1-Alkoxy-1,3-dien in Gegenwart eines geeigneten Katalysators, nämlich einer Brönstedsäure, zum entsprechenden kettenverlängerten δ-Alkoxy-α,β-ungesättigten Polyenaldehyd in Form dessen Ethylenacetals umsetzt, dieses Ethylenacetal zum entsprechenden Aldehyd hydrolysiert und schliesslich von diesem Aldehyd unter basischen oder sauren Bedingungen das δ-ständige Alkanol eliminiert, um den erwünschten konjugierten Polyenaldehyd zu erhalten. Nicht nur ist die Reaktion des 1-Alkoxy-1,3-diens mit dem Polyen-O,O-ethylenacetal neu, sondern es erfolgt dabei ein (soweit ersichtlich) ausschliesslicher Angriff an der γ-Stellung des Alkoxydiens. Das Entstehen des δ-Alkoxy-α,β-ungesättigten Polyen-O,O-ethylenacetals muss als völlig überraschend angesehen werden. Durch die sich der Hydrolyse anschliessende basen- oder säureninduzierte Elimination des Alkanols wird eine (konjugierte) C-C-Doppelbindung gebildet, ohne dass dazu ein phosphor-, silizium- oder schwefelhaltiges Reagens benötigt wird, was im Gegensatz zu der bisher auf diesem Gebiet üblicherweise verwendeten Methodik steht.

Demnach handelt es sich bei der vorliegenden Erfindung um ein Verfahren zur Herstellung eines Polyenaldehyds der allgemeinen Formel 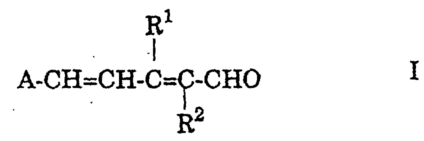 worin
- A: eine monovalente, gegebenenfalls methylsubstituierte, konjugierte Polyengruppe und
- R¹ und R²: jeweils Wasserstoff oder Methyl bedeuten,
wobei sich die Gruppe -CH=CH-C(R¹)=C(R²)-CHO in der Endstellung der konjugierten Kette der Gruppe A befindet,
das dadurch gekennzeichnet ist, dass man ein Polyen-O,O-ethylenacetal der allgemeinen Formel 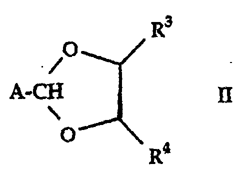 worin
- A: die oben angegebene Bedeutung besitzt, wobei sich in diesem Fall die Ethylenacetalgruppe 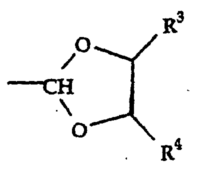 in der Endstellung der konjugierten Kette der Gruppe A befindet, und
- R³ und R⁴: jeweils Wasserstoff oder C₁₋₄-Alkyl bedeuten,
mit einem 1-Alkoxy-1,3-dien der allgemeinen Formel 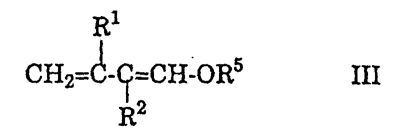 worin
- R¹ und R²: die oben angegebenen Bedeutungen besitzen und
- R⁵: C₁₋₆-Alkyl bedeutet,
in Gegenwart einer Brönstedsäure zur Verbindung der allgemeinen Formel 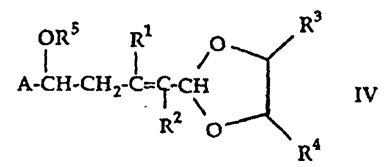 worin A, R¹, R², R³, R⁴ und R⁵ die oben angegebenen Bedeutungen besitzen,
umsetzt, die Verbindung der Formel IV hydrolysiert, und von der so erhaltenen Verbindung der allgemeinen Formel 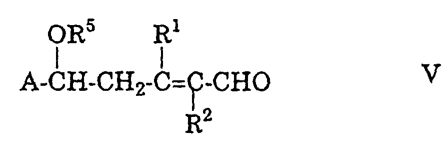 worin A, R¹, R² und R⁵ die oben angegebenen Bedeutungen besitzen, wobei sich in diesem Fall die Gruppe -CH(OR⁵)-CH₂-C(R¹)=C(R²)-CHO in der Endstellung der konjugierten Kette der Gruppe A befindet,
unter basischen oder sauren Bedingungen das Alkanol R⁵OH abspaltet.

Das erfindungsgemässe Verfahren kann im Prinzip bei allen oben erwähnten Polyen-O,O-ethylenacetalen der Formel II, welche am Ende der Polyenkette die Ethylenacetalgruppe aufweisen, Verwendung finden. Unter solchen Edukten befinden sich u.a. die folgenden Unterklassen [wobei die in der Carotinoid-Chemie übliche (mit einfachen Strichen) abgekürzte Darstellungsweise für die Strukturformeln benutzt wird]:

Alicyclisch-aliphatische Polyen-O,O-ethylenacetale, die hauptsächlich dem Gebiet der Carotinoide [als Ethylenacetale asymmetrischer Carotinoid-Aldehyde mit einem sechsgliedrigen (Cyclohexen-)Ring] angehören, der allgemeinen Formel 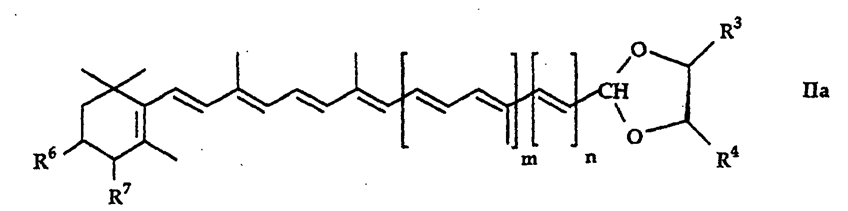 worin
- R³ und R⁴: die oben angegebenen Bedeutungen besitzen und
- R⁶ und R⁷: unabhängig voneinander Wasserstoff, eine gegebenenfalls geschützte Hydroxygruppe oder eine gegebenenfalls geschützte Oxogruppe,
- m: 0, 1, 2, 3 oder 4 und
- n: 0 oder 1 bedeuten,
welche nach Durchführung des erfindungsgemässen mehrstufigen Verfahrens in die entsprechenden alicyclisch-aliphatischen Polyenaldehyde der allgemeinen Formel 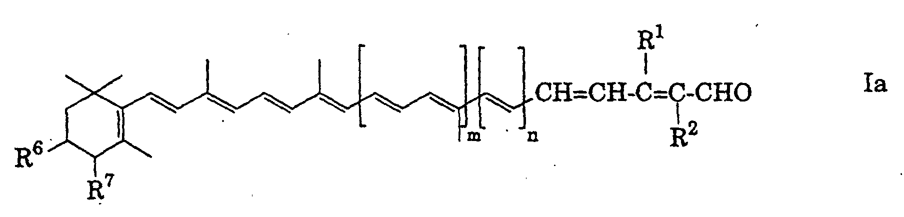 übergeführt werden; sowie

Aliphatische Polyen-O,O-ethylenacetale, die ebenfalls hauptsächlich dem Gebiet der Carotinoide (als Ethylenacetale offenkettiger asymmetrischer Carotinoid-Aldehyde) angehören, der allgemeinen Formel 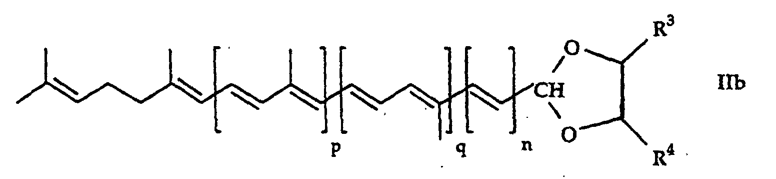 worin
- R³ und R⁴: die oben angegebenen Bedeutungen besitzen und
- p: 0, 1 oder 2,
- q: 0, 1, 2 oder 3 und
- n: 0 oder 1 bedeuten,
welche nach Durchführung des erfindungsgemässen mehrstufigen Verfahrens in die entsprechenden aliphatischen Polyenaldehyde der allgemeinen Formel 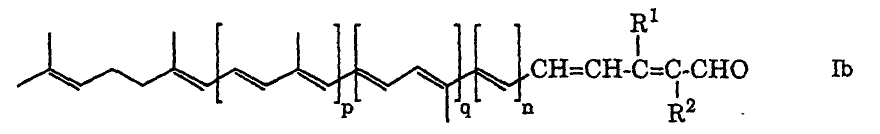 übergeführt werden.

Die Edukte der allgemeinen Formeln IIa und IIb können durch die allgemeine Formel II' umfasst werden: 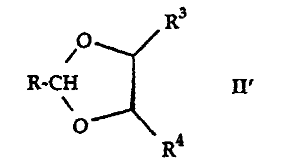 worin
- R: eine Gruppe (a) oder (b) 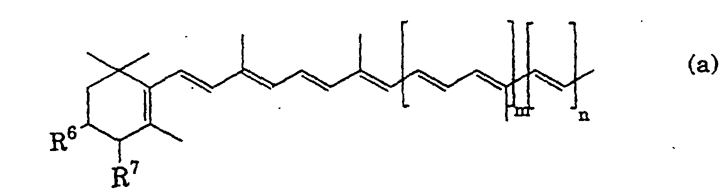 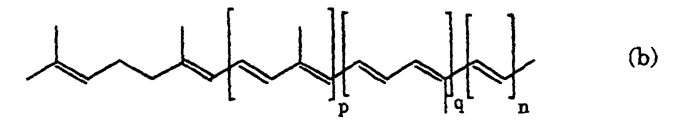 bedeutet und
- R³, R⁴, R⁶, R⁷, m, n, p und q: die oben angegebenen Bedeutungen besitzen.

Nach Durchführung des erfindungsgemässen mehrstufigen Verfahrens wird das Edukt der Formel II' in das entsprechende Produkt der Formel I' übergeführt:

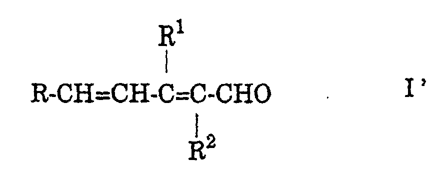

Die Formel I' umfasst dann die Formeln Ia und Ib.

Falls das Produkt der Formel I, insbesondere der Formel Ia, am Cyclohexenring eine oder zwei geschützte Gruppen (R⁶, R⁷) aufweist, kann man gewünschtenfalls die vorhandene(n) Schutzgruppe(n) abspalten, was einen weiteren Aspekt der vorliegenden Erfindung darstellt.

Im Rahmen der vorliegenden Erfindung umfasst der Ausdruck "C₁₋₄-Alkyl", "C₁₋₅-Alkyl" bzw. "C₁₋₆-Alkyl" geradkettige und verzweigte Gruppen, wie beispielsweise Methyl, Ethyl und Isobutyl. Analoges gilt für die Alkoxygruppen.

Der Ausdruck "geschützte Hydroxygruppe" umfasst übliche (besonders auf dem Gebiet der Carotinoide geläufige) geschützte Hydroxygruppen, insbesondere veretherte Hydroxygruppen und Acyloxygruppen. Bei den "veretherten Hydroxygruppen" handelt es sich beispielsweise um C₁₋₅-Alkoxygruppen, vorzugsweise Methoxy und Ethoxy; C₂₋₄-Alkoxyalkoxygruppen, vorzugsweise 1-Methoxy-1-methylethoxy; Arylalkoxygruppen, vorzugsweise Benzyloxy; Tetrahydropyranyloxy; und Tri(C₁₋₅alkyl)silyloxygruppen, vorzugsweise Trimethylsilyloxy. Die Acyloxygruppen umfassen insbesondere Alkanoyloxy- und Aroyloxygruppen mit bis zu 8 Kohlenstoffatomen, wie beispielsweise Formyloxy, Acetoxy, Propionyloxy und Benzoyloxy.

Der Ausdruck "geschützte Oxogruppe" umfasst ebenfalls übliche (besonders auf dem Gebiet der Carotinoide geläufige) geschützte Oxogruppen. Bevorzugt sind die acetalisierten Oxogruppen, insbesondere diejenigen, worin der Ausdruck geschützte Oxogruppe für zwei C₁₋₅-Alkoxygruppen (z.B. für zwei Methoxygruppen) oder für eine C₂₋₆-Alkylendioxygruppe (z.B. Ethylendioxy oder 2,3-Butylendioxy) steht. Ferner kann eine Oxogruppe auch als Enolether geschützt sein, und zwar vor allem im Falle von α-Hydroxyketonen (z.B. R⁶ und R⁷ bedeuten Hydroxy resp. Oxo oder umgekehrt), wobei die Veretherung des Endiols vorzugsweise auch durch Bildung eines cyclischen Acetals oder Ketals (z.B. mit Aceton zum Acetonid) erfolgen kann. Die Oxogruppe kann auch beispielsweise als ein Imin geschützt sein.

Die im Rahmen der vorliegenden Erfindung offenbarten Formeln von Polyenen umfassen jeweils isomere Formen, z.B. optisch aktive und cis/trans bzw. E/Z-Isomere, sowie Gemische hiervon, sofern nicht ausdrücklich anders erwähnt. Als Beispiel eines chiralen (optischen aktiven) Zentrums sei erwähnt das den Rest R⁶ oder R⁷ tragende Kohlenstoffatom, falls R⁶ bzw. R⁷ eine gegebenenfalls geschützte Hydroxygruppe bedeutet (siehe Formeln Ia und IIa). Was die E/Z-Isomerie anbelangt, so sind im allgemeinen die (all-E)-Isomeren der Edukte und der Produkte des erfindungsgemässen Verfahrens bevorzugt.

Der erste Verfahrensschritt des erfindungsgemässen Verfahrens wird zweckmässigerweise durchgeführt, indem man das Polyen-O,O-ethylenacetal der Formel II mit dem 1-Alkoxy-1,3-dien der Formel III in einem organischen Lösungsmittel bei Temperaturen im Bereich von etwa -60°C bis etwa +60°C, vorzugsweise im Temperaturbereich von etwa -20°C bis zur Raumtemperatur, und in Gegenwart einer Brönstedsäure umsetzt. Als organische Lösungsmittel eignen sich im allgemeinen alle aprotischen polaren oder unpolaren Lösungsmittel. Bevorzugte derartige Lösungsmittel sind niedere aliphatische und cyclische Kohlenwasserstoffe, z.B. Pentan, Hexan und Cyclohexan; niedere, halogenierte aliphatische Kohlenwasserstoffe, z.B. Methylenchlorid und Chloroform; niedere aliphatische und cyclische Ether, z.B. Diethylether, tert.Butylmethylether und Tetrahydrofuran; niedere aliphatische Nitrile, z.B. Acetonitril; sowie aromatische Kohlenwasserstoffe, z.B. Toluen. Das besonders bevorzugte Lösungsmittel ist Toluen. Beispiele der verwendbaren Brönstedsäuren sind p-Toluensulfonsäure, Methansulfonsäure, Trifluormethansulfonsäure, Schwefelsäure sowie Trifluoressigsäure. Diese werden im allgemeinen in katalytischen Mengen eingesetzt, und zwar zweckmässigerweise in einer Menge, die zwischen etwa 0,5 und 5 Molprozent bezogen auf die eingesetzte Menge des Polyen-O,O-ethylenacetals beträgt, und vorzugsweise im Molprozentbereich von 1% bis 2% liegt. Zudem verwendet man zweckmässigerweise etwa 1,05 bis etwa 2 Aequivalente 1-Alkoxy-1,3-dien pro Aequivalent Polyen-O,O-ethylenacetal, vorzugsweise etwa 1,2 bis etwa 1,4 Aequivalente. Ausserdem erfolgt die Umsetzung zweckmässigerweise bei Normaldruck, wobei im allgemeinen der Druck nicht kritisch ist.

Gewünschtenfalls könnte das Zwischenprodukt der Formel IV aus dem Reaktionsgemisch isoliert und anschliessend zur entsprechenden Verbindung der Formel V hydrolysiert werden. Allerdings erweist es sich als zweckmässiger, keine derartige Isolierung und nachfolgende Hydrolyse vorzunehmen, sondern unmittelbar nach Beendigung der Reaktion II + III das Zwischenprodukt im Reaktionsgemisch selber zu hydrolysieren, um in diesen Fällen zur Verbindung der Formel V zu gelangen. Die Hydrolyse kann geeigneterweise erfolgen, indem man zum Reaktionsgemisch eine Säure, vorzugsweise p-Toluensulfonsäure oder leicht verdünnte wässrige Essigsäure, gibt und das Gemisch anschliessend eine Weile, beispielsweise etwa 30 Minuten bis etwa 2 Stunden, rührt, und zwar zweckmässigerweise im Temperaturbereich von etwa 0°C bis zur Raumtemperatur.

Das Produkt der Formel V kann in an sich bekannter Weise vom Reaktionsgemisch isoliert und gewünschtenfalls gereinigt werden. Typischerweise wird das Gemisch mit Wasser vereinigt und das Ganze mit einem mit Wasser nicht mischbaren organischen Lösungsmittel, wie beispielsweise mit einem niederen Alkan, Dialkylether oder aliphatischen Ester, z.B. Hexan, tert.Butylmethylether bzw. Ethylacetat, extrahiert und die organische Phase mit Wasser und/oder Natriumbicarbonat- und/oder gesättigter wässriger Natriumchlorid-Lösung gewaschen, getrocknet und eingeengt. Das so isolierte und zumindest einigermassen gewaschene Rohprodukt kann dann gewünschtenfalls weitergereinigt werden, beispielsweise durch Säulenchromatographie, z.B. unter Verwendung von solchen Eluierungsmitteln als Hexan, Ethylacetat, Toluen oder Gemischen davon, oder (Um)Kristallisation, beispielsweise aus einem Alkohol, z.B. Methanol oder Ethanol. Alternativ, und oft bevorzugt, kann das beispielsweise in ein niederes Alkanol aufgenommene Rohprodukt unmittelbar im letzten Verfahrensschritt der vorliegenden Erfindung umgesetzt werden, und zwar im Rahmen eines "Durchprozesses" II + III → IV → V → I.

Was den letzten Verfahrensschritt, d.h. die Abspaltung des Alkanols R⁵OH von der Verbindung der Formel V, anbelangt, so sind Eliminierungen des Alkanols aus β-Alkoxyaldehyden oder δ-Alkoxy-α,β-ungesättigten Aldehyden unter Bildung der entsprechenden α,β-ungesättigten Aldehyde in der Fachliteratur bekannt und können unter verschiedenen Bedingungen durchgeführt werden. Beispielsweise wird im Rahmen bekannter baseninduzierter Eliminierungen als Base sehr oft 1,8-Diazabicyclo[5.4.0]undec-7-en verwendet, und zwar in einer Menge von etwa 2 bis 4 Aequivalenten bezogen auf die Menge eingesetzten Aldehyds. Es werden solche Bedingungen bei der bekannten Herstellung von Carotinoiden [siehe u.a. Bull. Chem. Soc. Japan 50, 1161 ff. (1977), ibid. 51, 2077 ff. (1978), Chem. Lett. 1975, 1201 ff. und Deutsche Offenlegungsschrift 2.701.489] und von Vitamin A (siehe u.a. Chem. Lett. 1975, 1201 ff.) verwendet. Auch Aluminiumoxid ist bei der Herstellung von Vitamin A durch Alkanolabspaltung verwendet worden [J. Gen. Chem. USSR 32, 63 ff. (1962)]. Als Beispiele von säureninduzierten Alkanolabspaltungen wird nochmals auf Bull. Chem. Soc. Japan 50 1161 ff. (1977) sowie auf J. Gen. Chem. USSR 30, 3875 ff. (1960) verwiesen, bei denen p-Toluensulfonsäure bzw. 85%ige Phosphorsäure als saurer Katalysator verwendet wird. Besonders bei der Herstellung von Carotinoiden ist für eine derartige Abspaltung das Puffersystem Natriumacetat/Essigsäure [Helv. Chem. Acta. 39, 249 ff. und 463 ff. (1956) und US-Patentschriften 2.827.481 und 2.827.482] oder Natriumformiat/ Ameisensäure [Synthesis 1981, 137 ff.] eingesetzt worden. Unter Heranziehung dieser und anderer einschlägiger Literatur ist der Fachmann ohne weiteres in der Lage, geeignete Reaktionsbedingungen für die erfolgreiche Durchführung des letzten Schrittes des erfindungsgemässen Verfahrens zu finden.

Darüber hinaus kann die Abspaltung des Alkanols R⁵OH auch nur mit katalytischen Mengen einer Base, d.h. mit weniger als einem Aequivalent bezogen auf ein Aequivalent der Verbindung der Formel V, durchgeführt werden. So wird der letzte Verfahrensschritt in diesem Fall zweckmässigerweise durchgeführt, indem man die in einem geeigneten organischen Lösungsmittel gelöste Verbindung der Formel V in Gegenwart einer katalytischen Menge Base unter Abspaltung des Alkanols R⁵OH zum entsprechenden Polyenaldehyd der Formel I umsetzt. Als organische Lösungsmittel eignen sich im allgemeinen protische, aprotische oder Gemische davon, wie beispielsweise Alkohole und Alkoholgemische; bzw. aromatische Kohlenwasserstoffe, z.B. Toluen; und niedere aliphatische Ester, z.B. Ethylacetat. Die Base kann anorganisch oder organisch sein, und es eignen sich im allgemeinen starke Basen, wie beispielsweise Alkalimetallalkoholate, z.B. Natriummethylat, Natriumethylat, Kaliummethylat, Kaliumethylat und Kalium-tert.butylat; Amine, z.B. Triethylamin, 1,5-Diazabicyclo[4.3.0]non-5-en und 1,8-Diazabicyclo[5.4.0]undec-7-en; sowie Alkalimetallhydroxide und - carbonate, insbesondere Natrium- und Kaliumhydroxid bzw. carbonat. Wie oben erwähnt, verwendet man zweckmässigerweise höchstens ein Aequivalent Base pro Aequivalent der Verbindung der Formel V, vorzugsweise etwa 0,05 bis etwa 0,3 Aequivalent. Die Umsetzung erfolgt geeigneterweise im Temperaturbereich von etwa -20°C bis etwa 100°C, vorzugsweise bei Temperaturen von etwa 0°C bis etwa 50°C. Ausserdem erfolgt die Umsetzung zweckmässigerweise bei Normaldruck, wobei im allgemeinen der Druck nicht kritisch ist.

Es hat sich als besonders vorteilhaft erwiesen, den letzten Verfahrensschritt unter Verwendung eines Natriumalkoxids als Base und des entsprechenden Alkanols als Lösungsmittel bei Temperaturen zwischen etwa -20°C und der Rückflusstemperatur des jeweiligen Reaktionsgemisches, vorzugsweise im Temperaturbereich von etwa 0°C bis etwa 40°C, durchzuführen. Dabei wird zweckmässigerweise im voraus entweder eine Lösung des Natriumalkoxids im Alkanol bereitgestellt, oder man stellt diese Lösung frisch aus metallischem Natrium und dem Alkanol her. Das Zusammenbringen der alkanolischen Lösung des Natriumalkoxids mit der vorzugsweise ebenfalls im voraus vorbereiteten Lösung der Verbindung der Formel V in (dem gleichen) Alkanol kann in beliebiger Reihenfolge und vorzugsweise bei Raumtemperatur erfolgen. Man rührt dann das Reaktionsgemisch, und die Reaktion ist normalerweise spätestens nach einer bis drei Stunden beendigt.

Ungeachtet der gewählten Prozedur des letzten Verfahrensschrittes kann das Produkt in an sich bekannter Weise vom Reaktionsgemisch isoliert und gereinigt werden. Im Falle der Verwendung eines basischen Katalysators beinhaltet die jeweilige Aufarbeitung normalerweise das Neutralisieren der verbleibenden Base, und zwar durch Zugabe einer organischen oder anorganischen Säure, wie beispielsweise einer Carbonsäure, z.B. Essigsäure, bzw. einer wässrigen Mineralsäure, z.B. verdünnter Schwefelsäure.

Im besonderen Fall der oben beschriebenen Prozedur mit dem Natriumalkoxid als Base wird nach Beendigung der Reaktion das Gemisch zweckmässigerweise auf Raumtemperatur oder sogar bis auf etwa 0°C abgekühlt und danach vorzugsweise mit wässriger Essigsäure neutralisiert. Die Auskristallisation des Produktes der Formel I kann auch noch durch weiteres Abkühlen gefördert werden. Nach dessen Isolierung, geeigneterweise durch Abfiltration, kann das Produkt gewaschen, beispielsweise mit Wasser und/oder wässrigem Alkohol, und schliesslich gegebenenfalls unter vermindertem Druck getrocknet werden. Gewünschtenfalls können solche weitere Methoden wie Säulenchromatographie und Umkristallisation eingesetzt werden, um zu einem noch reineren Produkt zu gelangen.

Im erhaltenen Produkt der Formel I gegebenenfalls vorhandene Schutzgruppen (R⁶ und/oder R⁷ als geschützte Hydroxy- oder Oxogruppe) können gewünschtenfalls nach an sich bekannten Methoden, z.B. durch Hydrolyse mit Säure oder Base, abgespalten werden.

In dem oben definierten erfindungsgemässen Verfahren bedeuten A bzw. R vorzugsweise eine Gruppe (a), in der R⁶ und R⁷ beide Wasserstoff und n 0 bedeuten, R¹ und R² vorzugsweise Wasserstoff resp. Methyl, und R³ und R⁴ beide vorzugsweise Wasserstoff.

Wie oben erwähnt, besteht bei der Durchführung des erfindungsgemässen Verfahrens gegenüber dem Stande der Technik (insbesondere den obenerwähnten Arbeiten von Nazarov und Krasnaya, Makin sowie Chemla et al.) der Vorteil darin, dass u.a. die Telomerbildung weitgehend unterdrückt wird. Obwohl bei dem erfindungsgemässen Verfahren die Telomerbildung als Folge der weiteren Reaktion der Verbindung der Formel IV mit dem 1-Alkoxy-1,3-dien der Formel III nicht immer vollständig unterdrückt werden kann, ist dies schliesslich weit weniger gravierend als erwartet. Die nach der zwischenstufigen Hydrolyse erfolgende Abspaltung des Alkanols R⁵OH von der Verbindung der Formel V kann nämlich ohne weiteres in Gegenwart eines in relativ kleiner Menge als Nebenprodukt mithergestellten Telomers, z.B. der Formel A-CH(OR⁵)-CH₂-C(R¹)=C(R²)-CH(OR⁵)-OH₂-O(R¹)=C(R²)-CHO (einmalige weitere Reaktion), erfolgen, wobei unter den speziell angewandten Reaktionsbedingungen auch eine analoge Alkanolabspaltung von dem Telomer stattfindet. Die letztere Abspaltung erfolgt jedoch unvollständig, indem praktisch nur die von der endständigen Aldehydgruppe am nächsten gelegene Alkoxygruppe OR⁵ (δ-Alkoxy) abgespalten wird. Das Ergebnis dieser unvollständigen Alkanolabspaltung vom Telomer besteht darin, dass das erwünschte Produkt der Formel I vom in diesem Stadium vorliegenden Nebenprodukt viel leichter entfernt werden kann als wenn alle Alkoxygruppen OR⁵ vom Telomer abgespalten würden. So bleibt nämlich das Nebenprodukt, welches noch einen oder mehrere Substituenten OR⁵ aufweist, in der Mutterlauge des Reaktionsgemisches, während das erwünschte Produkt auskristallisiert und demzufolge leicht zu entfernen ist, z.B. durch Filtration. Dass eben das Telomer nur den (jeweiligen) δ-ständigen Alkanol R⁵OH bei dem Abspaltungsschritt des erfindungsgemässen Verfahrens verliert, stellt ein völlig überraschendes Ergebnis dar.

Während einige der Edukte des erfindungsgemässen Verfahrens bekannt sind, sind andere aus zum Teil bekannten Vorstufen nach an sich bekannten Methoden herstellbar.

So können beispielsweise die neuen Polyen-O,O-ethylenacetale der Formel II sehr einfach auf bekannte allgemeine Weise durch Umsetzung des Polyenaldehyds der Formel A-CHO mit einem gegebenenfalls alkyl- oder dialkylsubstituierten 2-Niederalkoxy-1,3-dioxolan der allgemeinen Formel 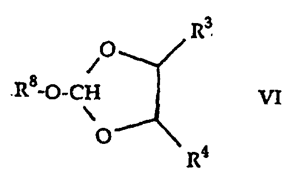 worin R³ und R⁴ die oben angegebenen Bedeutungen besitzen und R⁸ Niederalkyl, vorzugsweise C₁₋₄-Alkyl, insbesondere Methyl, bedeutet,
in Gegenwart einer katalytischen Menge einer Brönsted- oder Lewissäure, z.B. p-Toluensulfonsäure bzw. Zinkchlorid, hergestellt werden. Die Reaktion verläuft zweckmässigerweise in einem organischen Lösungsmittel, das geeigneterweise ein aprotisches polares oder unpolares Lösungsmittel ist. Bevorzugte derartige Lösungsmittel sind niedere aliphatische Kohlenwasserstoffe, z.B. Hexan; niedere, halogenierte aliphatische Kohlenwasserstoffe, z.B. Methylenchlorid und Chloroform; niedere aliphatische Ether, z.B. Diethylether; niedere aliphatische Ester, z.B. Ethylacetat; sowie aromatische Kohlenwasserstoffe, z.B. Benzen und Toluen. Die Umsetzung wird zweckmässigerweise im Temperaturbereich von etwa -20°C bis etwa +50°C durchgeführt und dauert in der Regel etwa 1 bis 4 Stunden. Viele Beispiele der allgemeinen Methodik sind aus der Literatur bekannt; vgl. u.a. J.A.C.S. 109, 1597 ff. (1987), ibid. 101, 2171 ff. (1979) und Synlett 1992, 766 ff.

Die gegebenenfalls alkyl- oder dialkylsubstituierten 2-Niederalkoxy-1,3-dioxolane selber können im voraus in situ aus dem entsprechenden ortho Ameisensäure-niederalkylester und dem entsprechenden gegebenenfalls alkyl- oder dialkylsubstituierten Ethylenglykol in Gegenwart der oben erwähnten Lewissäure hergestellt werden. Das dabei entstehende Alkanol R⁸OH muss sehr sorgfältig, vorzugsweise kontinuierlich, aus dem Gleichgewicht entfernt werden, und zwar zweckmässigerweise durch kontinuierliche Abdampfung unter vermindertem Druck.

Bekannte Polyen-O,O-ethylenacetale der Formel II sind beispielsweise Retinal-ethylenacetal (EP 0432021 A1), Retinal-propylenacetal (FR 2681784 A), Citral-ethylenacetal (EP 0268460 A1), Citral-propylenacetal (US-Patentschrift 4.832.059), Citral-2,3-dimethylethylenglykolacetal (Database Chemlist Chem. Abs. Service (ACS) XP 002095179) sowie Dehydrofarnesalethylenacetal (EP 0432021 A1).

Die Polyenaldehyde A-CHO ihrerseits sind entweder bekannt, insbesondere aus der Fachliteratur betreffend Carotinoide, oder - falls neu - können nach an sich bekannten einschlägigen Methoden hergestellt werden. So sind beispielsweise die Umsetzung von verschiedenen C₁₅-Wittigsalzen mit 2,7-Dimethyl-2,4,6-octatrien-1,8-dial (dem sogenannten "C₁₀-Dial") zu den entsprechenden Monoaldehyden, die Umsetzung von verschiedenen C₅-Wittigaldehyden mit langkettigen Polyenaldehyden ebenfalls zu solchen Monoaldehyden sowie die zweifache Umsetzung des C₁₀-Dialdehyds mit C₅oder C₁₀-Wittigaldehyden zu verschiedenen Dialdehyden aus dieser Literatur bekannt geworden. Die Lehrbücher "Carotenoids" (O. Isler, Birkhäuser Verlag Basel und Stuttgart, 1971), besonders dessen Kapitel VI und XII und die darin erwähnte weitere Literatur, und "Carotenoids, Volume 2 : Synthesis" (G. Britton, S. Liaaen-Jensen und H. Pfander, Birkhäuser Verlag Basel Boston Berlin, 1996), besonders dessen Kapitel III und VII, liefern viele nützliche Hinweise auf die Herstellung und das Vorkommen der bekannten Mono- und Dialdehyde. Falls Edukte eingesetzt werden, welche geschützte Hydroxy-, Oxo- bzw. Formylgruppen aufweisen, so können solche "geschützte" Edukte beispielsweise unmittelbar aus den entsprechenden ungeschützten nach an sich bekannten Methoden hergestellt werden.

Die 1-Alkoxy-1,3-diene der Formel III sind zum Teil bekannte Verbindungen; die restlichen (neuen) können aus bekannten Ausgangsmaterialien analog den bekannten Methoden hergestellt werden.

So ist beispielsweise das 1-Ethoxy-2-methyl-1,3-butadien (der Formel III, worin R¹ Wasserstoff, R² Methyl und R⁵ Ethyl bedeuten) aus der Literatur seit langem bekannt [siehe u.a. J.A.C.S. 91, 3281 ff. (1969), Bull. Soc. Chim. Fr. 1963, 1646 ff., sowie J. Gen. Chem. USSR 29, 3649 ff. (1959)] und wurde jeweils durch zweimalige Abspaltung von Ethanol aus 1,1,3-Triethoxy-2-methyl-butan hergestellt. Das Butan seinerseits kann durch eine seit langem bekannte Enoletherkondensation (siehe US-Patentschrift 2.165.962) aus den beiden gut zugänglichen Ausgangsmaterialien Acetaldehyd-diethylacetal und Ethyl-(1-propenyl)-ether hergestellt werden [siehe zudem J.A.C.S. 71, 3468 ff. (1949) sowie J. Gen. Chem. USSR 29, 3641 ff. (1959)]. Hierbei erwärmt man leicht etwa 2 bis 3 Aequivalente des Acetals pro Aequivalent Ethylpropenylether bis etwa 2 Stunden bei etwa 35°C mit etwa 0,2 Molprozent Bortrifluoridetherat als Katalysator ohne Lösungsmittel und erhält das erwünschte Butan in etwa 66%iger Ausbeute. Die anschliessende zweimalige Abspaltung von Ethanol aus dem 1,1,3-Triethoxy-2-methyl-butan kann gemäss dem einschlägigen Stand der Technik auf zwei verschiedenen Wegen realisiert werden, und zwar durch Abspaltung in der Flüssigphase (Bull. Soc. Chim. Fr. 1963, 1646 ff.) oder durch Abspaltung in der Gasphase [J. Gen. Chem. USSR 29, 3649 ff. (1959) und US-Patentschrift 2.573.678], vorzugsweise gemäss der letzteren Methode.

Auch das 1-Methoxy-2-methyl-1,3-butadien (der Formel III, worin R1 Wasserstoff und R² und R⁵ beide Methyl bedeuten) ist aus der Literatur [Japanische Patentpublikation (Kokai) 50891/1989] bekannt. Es kann beispielsweise analog der oben beschriebenen Herstellung von 1-Ethoxy-2-methyl-1,3-butadien ausgehend von Acetaldehyd-dimethylacetal und Methyl-(1-propenyl)-ether über das 1,1,3-Trimethoxy-2-methyl-butan hergestellt werden.

Uebersichtsartikel zur Herstellung der 1-Alkoxy-1,3-diene befinden sich in Russian Chem. Rev. 38, 237 ff. (1969) und in Pure and Appl. Chem. 47, 173 ff. (1976); für weitere Literatur über deren Herstellung durch Gasphasenkatalyse wird auf Lieb. Ann. Chem. 568, 1 ff. (1950), Can. J. Res. B 28, 689 ff. (1950), ibid. B 25, 118 ff. (1947) sowie Chem. Ber. 77, 108 ff. (1944) verwiesen.

Die Zwischenprodukte des erfindungsgemässen Verfahrens, d.h. die Verbindungen der allgemeinen Formel IV' (IV, worin das Symbol A durch R ersetzt ist), stellen einen weiteren Aspekt der vorliegenden Erfindung dar.

Unter diesen neuen Verbindungen befindet sich:
12'-Methoxy-11',12'-dihydro-8'-apo-β-carotinal-ethylenacetal.

Die Endprodukte des erfindungsgemässen Verfahrens, d.h. die Polyenaldehyde der allgemeinen Formel I, gehören grösstenteils dem Gebiet der Carotinoide an und finden entsprechende Verwendung, beispielsweise als Farbstoffe bzw. Pigmente für Lebensmittel, den Eidotter, die Integumente (insbesondere Haut, Ständer und Schnäbel) und/oder das subkutane Fett von Geflügel, das Fleisch und/oder die Integumente (insbesondere Haut, Schuppen und Schale) von Fischen und Crustaceen usw. Diese Verwendung kann nach an sich bekannten Methoden erfolgen, wie diese beispielsweise in der europäischen Patentpublikation Nr. 630.578 beschrieben ist.

Die Erfindung wird anhand der nachfolgenden Beispiele veranschaulicht:

### A. Herstellung der Polyen-O,O-ethylenacetale (Verbindungen der Formel II)

### Beispiel 1

### 12'-Apo-β-carotinal-ethylenacetal

In einem Rundkolben wurden 18,6 g (16,7 ml, 0,3 Mol) Ethylenglykol, 21,2 g (22 ml, 0,2 Mol) Orthoameinsensäure-trimethylester und 0,1 g p-Toluensulfonsäure-monohydrat vorgelegt. Das durch Umacetalisierung entstehende Methanol wurde am Rollverdampfer bei 30°C/100 mbar (10kPa) während 1 1/2 - 2 Stunden entfernt, und zwar bis zu einer Gewichtskonstanz von 25 g.

Die resultierende wasserklare Lösung wurde nun zu einer Suspension von 35,1 g (0,1 Mol) 12'-Apo-β-carotinal in 350 ml Hexan gegeben. Man rührte das Reaktionsgemisch bei Raumtemperatur, wobei sich das Edukt langsam löste. Nach etwa einer Stunde begann das Produkt auszukristallisieren. Nach insgesamt 3 1/2 Stunden gab man 0,5 ml Triethylamin zu, leerte das Gemisch auf 100 ml Wasser, extrahierte es mit 600 ml Diethylether und wusch die Lösung mit 100 ml gesättigter Natriumchloridlösung. Nach Trocknung mit wasserfreiem Magnesiumsulfat wurde filtriert und eingeengt. Dies ergab 39,5 g Rohprodukt als gelbe Nadeln, welche man in 600 ml Diethylether löste, mit 350 ml Methanol versetzte und am Rollverdampfer bei 200 mbar (20kPa) wieder vom Ether befreite. Die dabei entstehende eiskalte Suspension wurde genutscht und die Kristalle mit 300 ml eiskaltem Methanol gewaschen. Achtzehnstündige Trocknung bei Raumtemperatur/0,1 mbar (10Pa) ergab 32,7 g (83%) 12'-Apo-β-carotinal-ethylenacetal als hellgelbe Nadeln mit Smp. 128°C; Gehalt nach HPLC: 99,3%; UV (Hexan): 392 nm (log ε=4,93), 376 nm (log ε=4,94); IR (kein CHO); MS: 394 (M⁺, 100); ¹H-NMR (400 MHz, CDCl₃): 3,9-4,1 (2m, je 2H, OCH₂CH₂O), 5,19 (s, 1H, OCHO), kein CHO

| | | | |
|---|---|---|---|
| Mikroanalyse | Ber. | C 82,18% | H 9,71% |
| | Gef. | C 82,38% | H 9,78% |

### Beispiel 2

### 8'-Apo-β-carotinal-ethylenacetal

In einem Rundkolben wurden 3,7 g (60 mmol) Ethylenglykol, 4,25 g (40 mmol) Orthoameisensäure-trimethylester und 20 mg p-Toluensulfonsäure-monohydrat vorgelegt. Das durch Umacetalisierung entstehende Methanol wurde am Rollverdampfer bei 30°C/100 mbar (10kPa) während 2 1/2 Stunden entfernt, und zwar bis zu einer Gewichtskonstanz von 4,4 g.

Die resultierende Lösung wurde nun zu 4,17 g (10 mmol) 8'-Apo-β-carotinal in 50 ml Toluen gegeben. Man rührte das Reaktionsgemisch bei Raumtemperatur 6 Stunden lang. Nun wurde mit 0,5 ml Triethylamin neutralisiert, mit 100 ml Diethylether verdünnt, mit je 50 ml Wasser und 50 ml gesättigter Natriumchloridlösung gewaschen über wasserfreien Magnesiumsulfat getrocknet, filtriert und am Rollverdampfer eingeengt. Dies ergab 5,0 g dunkelrote, schmierige Kristalle, welche man aus 20 ml Ethylacetat (40°C) und 50 ml Methanol umkristallisierte (etwa 16 Stunden bei 0°C).

Auf diese Weise erhielt man 2,81 g (61%) 8'-Apo-β-carotinal-ethylenacetal als rötliche Kristalle mit Smp. 135°C. Eine analytische Probe besass Smp. 136°C; Gehalt nach HPLC: 99 %; UV (Cyclohexan/3 % CHCl₃): 454 nm (log ε=5,04), 443 nm (log ε=5,08); MS: 460 (M⁺, 100); IR (kein CHO); ¹H-NMR (400 MHz, CDCl₃): 3,9-4,1 (2m, je 2H, OCH₂CH₂O), 5,19 (s, 1H, OCHO);

| | | | |
|---|---|---|---|
| Mikroanalyse | Ber. | C83,43% | H 9,63% |
| | Gef. | C83,14% | H 9,45% |

### B. Herstellung der Verbindungen der Formeln IV und V

### Beispiel 3

### 12'-Methoxy-11',12'-dihydro-8'-apo-β-carotinal-ethylenacetal

1,97 g (5 mmol) 12'-Apo-β-carotinal-ethylenacetal wurden in 30 ml Hexan suspendiert und bei 0°C mit 1,1 g (11 mmol) 1-Methoxy-2-methyl-1,3-butadien in Gegenwart von 10 mg p-Toluensulfonsäure-monohydrat umgesetzt. Nach 2 1/2 Stunden neutralisierte man das Gemisch mit 0,1 ml Triethylamin, verdünnte es mit 30 ml Hexan, wusch es mit je 25 ml Wasser, gesättigter Natriumbicarbonatlösung und gesättigter Natriumchlorid-lösung, trocknete es über wasserfreiem Magnesiumsulfat und engte die Lösung am Rollverdampfer ein. Dies ergab 2,8 g zähflüssiges, dunkelrot gefärbtes Oel, welches man an 140 g Kieselgel (0,04-0,063 mm) mit Toluen/Hexan (95:5) chromatographierte.

Auf diese Weise erhielt man 0,49 g (20%) 12'-Methoxy-11',12'-dihydro-8'-apo-β-carotinal-ethylenacetal als (9' E/Z) -Isomerengemisch; ¹H-NMR (400 MHz, CDCl₃): 3,9-4,0 (2 m, je 2H, OCH₂CH₂O), 5,1(s, 1H, OCHO), kein CHO; MS 492 (M⁺, 100).

### Beispiel 4

### 12'-Methoxy-11',12'-dihydro-8'-apo-β-carotinal (Durchprozess aus 12'-Apo-β-carotinal-ethylenacetal und l-Methoxy-2-methyl-1.3-butadien über 12'-Methoxy-11',12'-dihydro-8'-apo-β-carotinal-ethylenacetal)

1,97 g (5 mmol) 12'-Apo-β-carotinal-ethylenacetal wurden mit 0,79 g (8 mmol) 1-Methoxy-2-methyl-1,3-butadien in 30 ml Toluen in Gegenwart von 19 mg (2 Mol%) p-Toluensulfonsäure-monohydrat bei -20°C umgesetzt. Nach 6 1/2 Stunden bei -20°C gab man zur Hydrolyse 10 ml 90%ige wässrige Essigsäure zu und rührte das Gemisch etwa 30 Minuten bei Raumtemperatur. Dann wurde das Gemisch je zweimal mit 20 ml Wasser, gesättigter Natriumbicarbonatlösung und gesättigter Natriumchloridlösung gewaschen, über wasserfreiem Magnesiumsulfat getrocknet und am Rollverdampfer eingeengt. Dies ergab 2,7 g rohes 12'-Methoxy-11',12'-dihydro-8'-apo-β-carotinal als oranges Oel. Chromatographie an 135 g Kieselgel (0,04-0,063 mm) mit Toluen/Ethylacetat (19:1) ergab 1,2 g (54%) 12'-Methoxy-11',12'dihydro-8'-apo-β-carotinal als oranges, zähflüssiges Oel. Reinheit nach HPLC: 95%; IR (Film): 1690 cm⁻¹(CHO); MS: 448 (M⁺, 100); ¹H-NMR (400 MHz, CDCl₃): 3,2 (s, 3H, OCH₃), 3,67 (t, 1H, CH-O), 9,40 (s, 1H, CHO).

### Beispiel 5

### 8'-Methoxy-7',8'-dihydro-4'-apo-β-carotinal (Durchprozess aus 8'-Apo-β-carotinal-ethylenacetal und 1-Methoxy-2-methyl-1,3-butadien über 8'-Methoxy-7',8'-dihydro-4'-apo-β-carotinal-ethylenacetal)

In einem mit Magnetrührer und Argonbegasung ausgestatteten 100 ml-Rundkolben wurden 3,35 g (7,5 mmol) 8'-Apo-β-carotinal-ethylenacetal mit 1,3 g (13,5 mmol) 1-Methoxy-2-methyl-1,3-butadien in 50 ml Toluen in Gegenwart von 30 mg p-Toluensulfonsäure-monohydrat bei -10°C umgesetzt. Nach 3 Stunden bei -10°C wurden zur Hydrolyse des intermediär entstehenden 8'-Methoxy-7',8'-dihydro-4'-apo-β-carotinal-ethylenacetal 15 ml 90%ige wässrige Essigsäure zugegeben. Das Gemisch wurde 2 Stunden bei Raumtemperatur gerührt und wie in Beispiel 4 beschrieben aufgearbeitet und chromatographiert. Dies ergab 2,4 g rohes 8'-Methoxy-7',8'-dihydro-4'-apo-β-carotinal, welches man in 30 ml Methanol bei 45°C digeriert, auf 0°C abkühlte, abfiltrierte und trocknete.

Auf diese Weise erhielt man 1,45 g (36%) 8'-Methoxy-7',8'-dihydro-4'-apo-β-carotinal als orangen Feststoff mit Smp. 152-3°C; Reinheit nach HPLC: 97%; UV(Cyclohexan/2% CHCl₃): 457 nm (log ε=5,03), 430 nm (log ε=5,08); ¹H-NMR (400 MHz, CDCl₃): 3,15 (s, 3H, OCH₃); 9,35 (s, 1H, CHO); MS: 514 (M⁺, 100).

### C. Herstellung der Polyenaldehyde der Formel I aus den Verbindungen der Formel V oder II und III (Durchprozess)

### Beispiel 6

### 8'-Apo-β-carotinal

In einem 50 ml Rundkolben wurden 1,16 g (2,6 mmol) 12'-Methoxy-11',12'-dihydro-8'-apo-β-carotinal in 15 ml Ethanol gelöst und die Lösung bei Raumtemperatur mit 0,1 ml (0,5 mmol) 5,4 M-Natriummethylatlösung in Methanol versetzt, wobei die Lösung sofort dunkel wurde und das Produkt langsam auskristallisierte. Nach 30 Minuten wurde mit 0,2 ml Essigsäure neutralisiert und 1,5 ml Wasser zugegeben. Dann wurde abgekühlt, abgenutscht und je einmal mit 2 ml Ethanol/Wasser (19:1) bei 0°C, 2 ml Wasser und 2 ml Ethanol/Wasser (19:1) bei 0°C gewaschen. Das Filtergut wurde 3 Stunden bei 50°C/0,1 mbar (10Pa) getrocknet.

Auf diese Weise erhielt man 0,80 g (74%) 8'-Apo-β-carotinal als blauviolette Kristalle mit Smp. 135,5-136,5°C; Gehalt nach HPLC: 97,7%.

### Beispiel 7

### 8'-Apo-β-carotinal (Durchprozess aus 12'-Apo-β-carotinal-ethylenacetal und 1-Methoxy-2-methyl-1.3-butadien über 12'-Methoxy-11',12'-dihydro-8'-apo-β-carotinal-ethylenacetal und 12'-Methoxy-11',12'-dihydro-8'-apo-β-carotinal

Zu einer Lösung von 5,91 g (15 mmol) 12'-Apo-β-carotinal-ethylenacetal und 2,36 g (24 mmol) 1-Methoxy-2-methyl-1,3-butadien in 90 ml Toluen wurden bei -20°C 57 mg (2 mol%) p-Toluensulfonsäure-monohydrat gegeben. Nach 4 1/2 Stunden wurden zur Hydrolyse 30 ml 90%ige wässrige Essigsäure zugegeben, die Kühlung entfernt und 2 Stunden bei Raumtemperatur gerührt. Die Lösung wurde nun zweimal mit je 60 ml Wasser und 30 ml gesättigter Natriumbicarbonatlösung und einmal mit 30 ml gesättigter Natriumchloridlösung gewaschen, über wasserfreiem Magnesiumsulfat getrocknet und eingeengt. Dies ergab 6,8 g rohes 12'-Methoxy-11',12'-dihydro-8'-apo-β-carotinal als oranges zähes Oel. Dieses Oel wurde nun bei Raumtemperatur in 100 ml Ethanol gelöst und unter Argon mit etwa 0,3 ml (16 mmol, 11 mol%) einer 5,4 M-Natriummethylatlösung versetzt, wobei die Lösung sofort dunkel wurde und das Produkt auskristallisierte. Nach 40 Minuten wurde auf 0°C abgekühlt, mit 0,6 ml Essigsäure neutralisiert und 4 ml Wasser zugetropft. Nun wurde filtriert, mit 10 ml Ethanol/Wasser (19:1) bei 0°C, zweimal mit je 10 ml Wasser und zweimal mit je 10 ml Ethanol/Wasser (19:1) bei 0°C gewaschen. Nach zweistündiger Trocknung bei 50°C und 0,1 mbar (10 Pa) erhielt man 4,02 g (63%) 8'-Apo-β-carotinal als blauviolette Kristalle mit Smp. 129-131°C. Gehalt nach HPLC 97,6%.

Zur weiteren Reinigung wurde das obige Produkt (4,02 g) in 70 ml Aceton etwa 10 Minuten unter Rückfluss gelöst. Zur Lösung gab man dann durch den Kühler unter Rückfluss und starkem Rühren 2,5 ml Wasser tropfenweise zu, was eine Kristallisation auslöste, und dann wurde langsam auf 0°C abgekühlt. Nachdem im Eisbad etwa 2 Stunden gerührt worden war, filtrierte man den Niederschlag ab und wusch ihn zweimal mit je 10 ml, total 20 ml, Ethanol/Wasser (9:1) bei 0°C, zweimal mit je 25 ml, total 50 ml, Wasser und schliesslich dreimal mit je 5 ml, total 15 ml, Ethanol/Wasser (9:1) bei 0°C. Nach Trocknung unter Hochvakuum bei Raumtemperatur erhielt man 3,40 g (54%ige Ausbeute) reines 8'-Apo-β-carotinal als blauviolette metallisch glänzende Kristalle mit Smp. 138-138,5°C und einem Gehalt nach HPLC von 99,5%.

Aus der Mutterlauge konnte durch Chromatographie an Kieselgel [Toluen/Ethylacetat (19:1)] nochmals 0,3 g (etwa 5%) 8'-Apo-β-carotinal mit Smp. 132°C erhalten werden. Insgesamt erhielt man 3,7 g (etwa 59%) 8'-Apo-β-carotinal.

### Beispiel 8

### 4'-Apo-β-carotinal

In einem 100 ml-Rundkolben wurden 1,30 g (2,37 mmol) 8'-Methoxy-7',8'dihydro-4'-apo-β-carotinal in 30 ml Methanol/Ethylacetat (1:1) vorgelegt, mit 0,1 ml (0,5 mmol) einer 5,4 M-Natriummethylatlösung in Methanol versetzt und 2 Stunden bei 50°C gerührt. Die anfallende dunkle Suspension wurde nun abgekühlt (0°C), abfiltriert mit Methanol/Wasser (9:1) und Methanol (0°C) gewaschen und unter Hochvakuum getrocknet. Dies ergab 1,0 g (81%) 4'-Apo-β-carotinal als violette Kristalle mit Smp. 155°C. Gehalt nach HPLC: 96%; UV(Cyclohexan/2% CHCl₃): 491 nm (log ε=5,11); ¹H-NMR (400 MHz, CDCl₃): 9.45 ppm (CHO). MS: 482 (M⁺, 100%); IR (Nujol): 1680 cm⁻¹.

## Patentansprüche

1. Verfahren zur Herstellung eines Polyenaldehyds der allgemeinen Formel 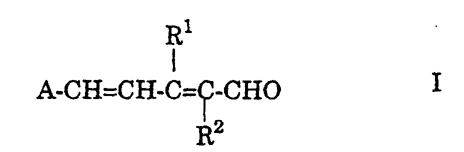 worin
A eine monovalente, gegebenenfalls methylsubstituierte, konjugierte Polyengruppe und
R¹ und R² jeweils Wasserstoff oder Methyl bedeuten,
wobei sich die Gruppe -CH=CH-C(R¹)=C(R²)-CHO in der Endstellung der konjugierten Kette der Gruppe A befindet,
**dadurch gekennzeichnet, dass** man ein Polyen-O,O-ethylenacetal der allgemeinen Formel 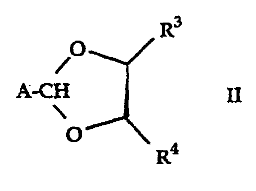 worin
A die oben angegebene Bedeutung besitzt, wobei sich in diesem Fall die Ethylenacetalgruppe 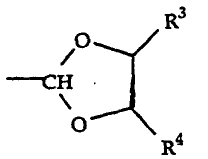 in der Endstellung der konjugierten Kette der Gruppe A befindet, und
R³ und R⁴ jeweils Wasserstoff oder C₁₋₄-Alkyl bedeuten,
mit einem 1-Alkoxy-1,3-dien der allgemeinen Formel 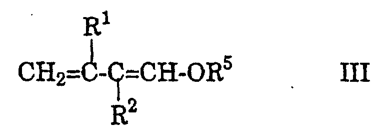 worin
R¹ und R² die oben angegebenen Bedeutungen besitzen und
R⁵ C₁₋₆-Alkyl bedeutet,
in Gegenwart einer Brönstedsäure zur Verbindung der allgemeinen Formel 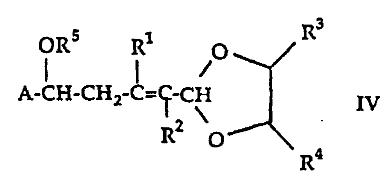 worin A, R¹, R², R³, R⁴ und R⁵ die oben angegebenen Bedeutungen besitzen,
umsetzt, die Verbindung der Formel IV hydrolysiert, und von der so erhaltenen Verbindung der allgemeinen Formel 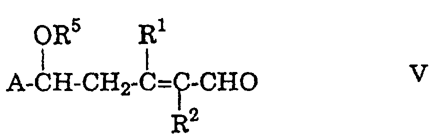 worin A, R¹, R² und R⁵ die oben angegebenen Bedeutungen besitzen, wobei sich in diesem Fall die Gruppe -CH(OR⁵)-CH₂-C(R¹)=C(R²)-CHO in der Endstellung der konjugierten Kette der Gruppe A befindet,
unter basischen oder sauren Bedingungen das Alkanol R⁵OH abspaltet.

2. Verfahren nach Anspruch 1, **dadurch gekennzeichnet, dass** als Polyen-O,O-ethylenacetal eine Verbindung der allgemeinen Formel 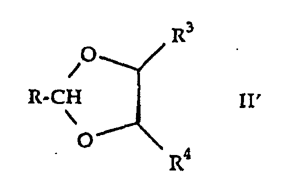 worin
R eine Gruppe (a) oder (b) 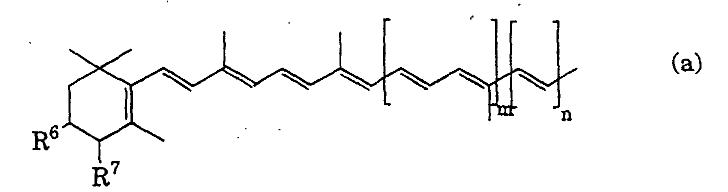 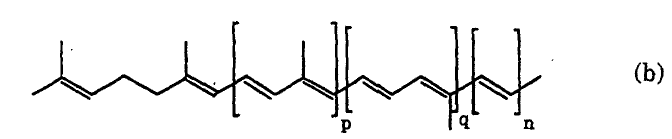 bedeutet und
R³ und R⁴ die in Anspruch 1 angegebenen Bedeutungen besitzen,
R⁶ und R⁷ unabhängig voneinander Wasserstoff, eine gegebenenfalls geschützte Hydroxygruppe oder eine gegebenenfalls geschützte Oxogruppe,
m 0, 1, 2, 3 oder 4 ,
n 0 oder 1,
p 0, 1 oder 2, und
q 0,1, 2 oder 3 bedeuten,
nach Durchführung des erfindungsgemässen mehrstufigen Verfahrens in den entsprechenden alicyclisch-aliphatischen Polyenaldehyd der allgemeinen Formel 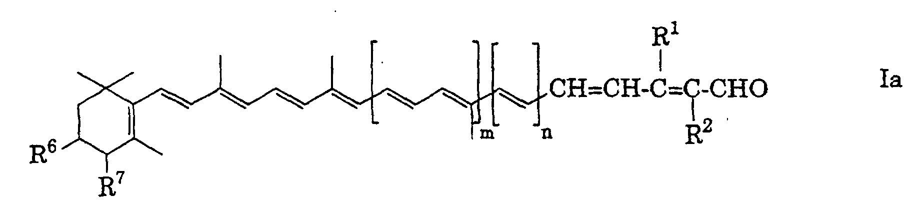 bzw. in den entsprechenden aliphatischen Polyenaldehyd der allgemeinen Formel 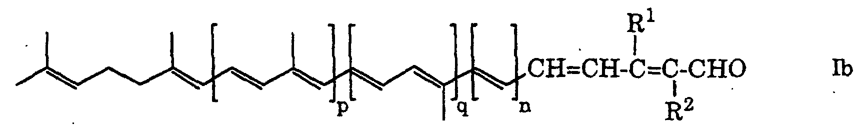 übergeführt wird, und dass man im Falle des Vorhandenseins einer Gruppe (a) im Produkt der Formel I gewünschtenfalls eine allfällig vorhandene Schutzgruppe abspaltet.

3. Verfahren nach Anspruch 2, **dadurch gekennzeichnet, dass** R eine Gruppe (a) bedeutet, in der R⁶ und R⁷ beide Wasserstoff und n 0 bedeuten.

4. Verfahren nach einem der Ansprüche 1 bis 3, **dadurch gekennzeichnet, dass** als Brönstedsäure p-Toluensulfonsäure, Methansulfonsäure, Trifluormethansulfonsäure, Schwefelsäure oder Trifluoressigsäure verwendet wird.

5. Verfahren nach einem der Ansprüche 1 bis 4, **dadurch gekennzeichnet, dass** die Brönstedsäure in einer katalytischen Menge eingesetzt wird, welche zwischen etwa 0,5 und 5 Molprozent bezogen auf die eingesetzte Menge des Polyen-O,O-ethylenacetals der Formel II bzw. II' beträgt.

6. Verfahren nach einem der Ansprüche 1 bis 5, **dadurch gekennzeichnet, dass** man etwa 1,05 bis etwa 2 Aequivalente 1-Alkoxy-1,3-dien pro Aequivalent Polyen-O,O-ethylenacetal umsetzt.

7. Verfahren nach einem der Ansprüche 1 bis 6, **dadurch gekennzeichnet, dass** man das Polyen-O,O-ethylenacetal der Formel II bzw. II' mit dem 1-Alkoxy-1,3-dien der Formel III in einem organischen Lösungsmittel bei Temperaturen im Bereich von etwa -60°C bis etwa +60°C umsetzt, wobei als organisches Lösungsmittel ein niederer aliphatischer oder cyclischer Kohlenwasserstoff, ein niederer halogenierter aliphatischer Kohlenwasserstoff, ein niederer aliphatischer oder cyclischer Ether, ein niederes aliphatisches Nitril oder ein aromatischer Kohlenwasserstoff verwendet wird.

8. Verfahren nach Anspruch 7, **dadurch gekennzeichnet, dass** als organisches Lösungsmittel Pentan, Hexan, Cyclohexan, Methylenchlorid, Chloroform, Diethylether, tert.Butylmethylether, Tetrahydrofuran, Acetonitril oder Toluen verwendet wird und die Umsetzung im Temperaturbereich von etwa -20°C bis zur Raumtemperatur erfolgt.

9. Verfahren nach einem der Ansprüche 1 bis 8, **dadurch gekennzeichnet, dass** man unmittelbar nach Beendigung der Reaktion des Polyen-O,O-ethylenacetals der Formel II bzw. II' mit dem 1-Alkoxy-1,3-dien der Formel III das daraus resultierende Zwischenprodukt im Reaktionsgemisch selber hydrolysiert, indem man zum Reaktionsgemisch eine wässrige Lösung einer schwachen Säure gibt und das Gemisch anschliessend im Temperaturbereich von etwa 0°C bis zur Raumtemperatur rührt.

10. Verfahren nach Anspruch 9, **dadurch gekennzeichnet, dass** als wässrige Lösung einer schwachen Säure leicht verdünnte wässrige Essigsäure verwendet wird.

11. Verfahren nach einem der Ansprüche 1 bis 10, **dadurch gekennzeichnet, dass** man die Abspaltung des Alkanols R⁵OH von der Verbindung der Formel V durchführt, indem man die in einem organischen Lösungsmittel gelöste Verbindung der Formel V in Gegenwart einer katalytischen Menge einer Base, wie beispielsweise eines Alkalialkoholats, eines Amins, eines Alkalimetallhydroxids oder eines Alkalimetallcarbonats umsetzt.

12. Verfahren nach Anspruch 11, **dadurch gekennzeichnet, dass** als Base Natriummethylat, Natriumethylat, Kaliummethylat, Kaliumethylat, Kaliumtert.butylat, Triethylamin, 1,5-Diazabicyclo [4.3.0]non-5-en, 1,8-Diazobicyclo[5.4.0]undec-7-en, Natrium- oder Kaliumhydroxid oder Natrium- oder Kaliumcarbonat verwendet wird.

13. Verbindungen der allgemeinen Formel 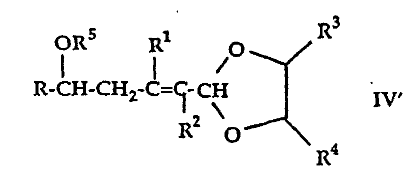 worin
R eine Gruppe (a) oder (b) 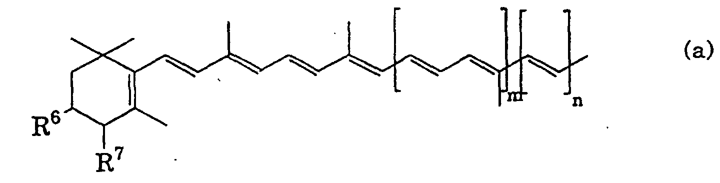 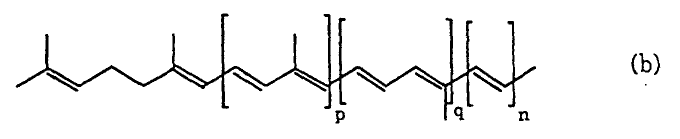 bedeutet und
R¹ und R² jeweils Wasserstoff oder Methyl,
R³ und R⁴ jeweils Wasserstoff oder C₁₋₄-Alkyl,
R⁵ C₁₋₆-Alkyl,
R⁶ und R⁷ unabhängig voneinander Wasserstoff, eine gegebenenfalls geschützte Hydroxygruppe oder eine gegebenenfalls geschützte Oxogruppe,
m 0, 1, 2, 3 oder 4 ,
n 0 oder 1,
p 0, 1 oder 2, und
q 0, 1, 2 oder 3 bedeuten.

14. Eine Verbindung nach Anspruch 13,
12'-Methoxy-11',12'-dihydro-8'-apo-β-carotinal-ethylenacetal.

## Claims

1. A process for the manufacture of a polyene aldehyde of the general formula 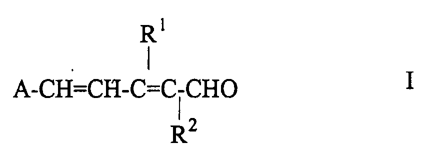 wherein
A signifies a monovalent, optionally methyl-substituted, conjugated polyene group and
R¹ and R² each signify hydrogen or methyl,
with the -CH=CH-C(R¹)=C(R²)-CHO group being situated at the terminal position of the conjugated chain of group A,
which process comprises reacting a polyene O,O-ethylene acetal of the general formula 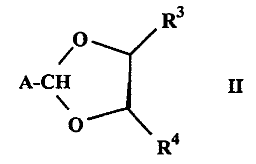 wherein
A has the significance given above, with in this case the ethylene acetal group 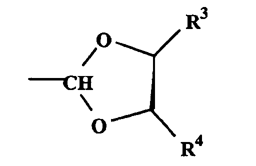 being situated at the terminal position of the conjugated chain of group A, and
R³ and R⁴ each signify hydrogen or C₁₋₄-alkyl,
with a 1-alkoxy-1,3-diene of the general formula 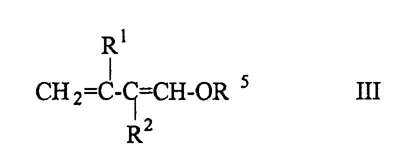 wherein
R¹ and R² have the significances given above and
R⁵ signifies C₁₋₆-alkyl,
in the presence of a Brönsted acid to give the compound of the general formula 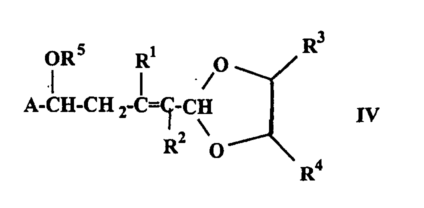 wherein A, R¹, R², R³, R⁴ and R⁵ have the significances given above,
hydrolyzing the compound of formula IV and cleaving off the alkanol R⁵OH under basic or acidic conditions from the thus-obtained compound of the general formula 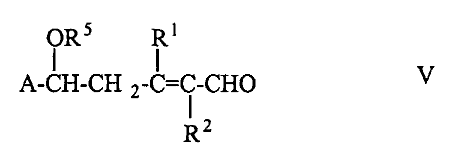 wherein A, R¹, R² and R⁵ have the significances given above, with in this case the -CH(OR⁵)-CH₂-C(R¹)=C(R²)-CHO group being situated at the terminal position of the conjugated chain of group A.

2. A process according to claim 1, wherein the polyene O,O-ethylene acetal used is a compound of the general formula 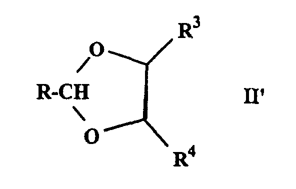 wherein
R signifies a group (a) or (b) 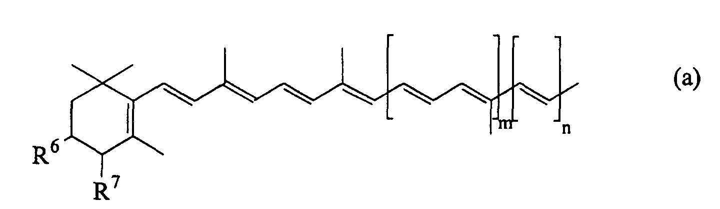 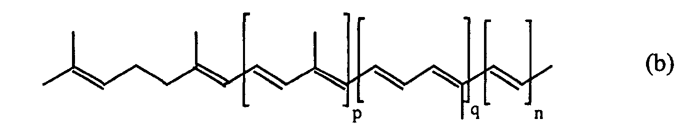
R³ and R⁴ have the significances given in claim 1,
R⁶ and R⁷ each independently signify hydrogen, an optionally protected hydroxy group or an optionally protected oxo group,
m signifies 0, 1, 2, 3 or 4,
n signifies 0 or 1,
p signifies 0, 1 or 2 and
q signifies 0, 1,2 or 3,
which after carrying out the multistage process in accordance with the invention is converted into the corresponding alicyclic-aliphatic polyene aldehyde of the general formula 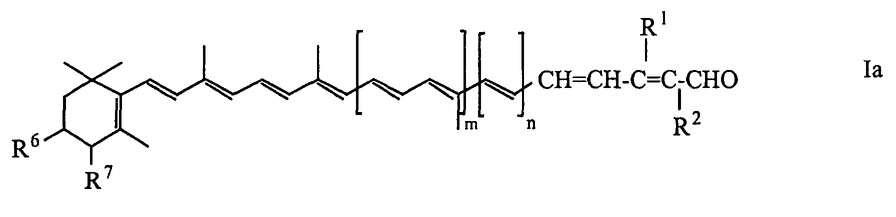 or into the corresponding aliphatic polyene aldehyde of the general formula 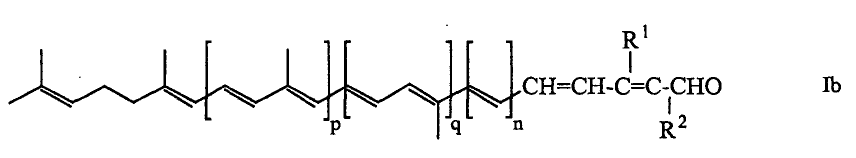 and, where a group (a) is present in the product of formula I, a protecting group which may be present is cleaved off, if desired.

3. A process according to claim 2, wherein R signifies a group (a) in which R⁶ and R⁷ both signify hydrogen and n signifies 0.

4. A process according to any one of claims 1 to 3, wherein p-toluenesulphonic acid, methanesulphonic acid, trifluoromethanesulphonic acid, sulphuric acid or trifluoroacetic acid is used as the Bronsted acid.

5. A process according to any one of claims 1 to 4, wherein the Brönsted acid is used in a catalytic amount which is between about 0.5 and 5 mol percent based on the amount of the polyene O,O-ethylene acetal of formula II or II' which is used.

6. A process according to any one of claims 1 to 5, wherein about 1.05 to about 2 equivalents of 1-alkoxy-1,3-diene are reacted per equivalent of polyene O,O-ethylene acetal.

7. A process according to any one of claims 1 to 6, wherein the polyene O,O-ethylene acetal of formula II or II' is reacted with the 1-alkoxy-1,3-diene of formula III in an organic solvent at temperatures in the range of about -60°C to about +60°C, with a lower aliphatic or cyclic hydrocarbon, a lower halogenated aliphatic hydrocarbon, a lower aliphatic or cyclic ether, a lower aliphatic nitrile or an aromatic hydrocarbon being used as the organic solvent.

8. A process according to claim 7, wherein pentane, hexane, cyclohexane, methylene chloride, chloroform, diethyl ether, tert.butyl methyl ether, tetrahydrofuran, acetonitrile or toluene is used as the organic solvent and the reaction is effected in the temperature range of about -20°C to room temperature.

9. A process according to any one of claims 1 to 8, wherein immediately after completion of the reaction of the polyene O,O-ethylene acetal of formula II or II' with the 1-alkoxy-1,3-diene of formula III the intermediate resulting therefrom is itself hydrolyzed in the reaction mixture by adding to the reaction mixture an aqueous solution of a weak acid and subsequently stirring the mixture in the temperature range of about 0°C to room temperature.

10. A process according to claim 9, wherein slightly diluted aqueous acetic acid is used as the aqueous solution of a weak acid.

11. A process according to any one of claims 1 to 10, wherein the cleavage of the alkanol R⁵OH from the compound of formula V is carried out by reacting the compound of formula V, dissolved in an organic solvent, in the presence of a catalytic amount of a base such as, for example, an alkali alcoholate, an amine, an alkali metal hydroxide or an alkali metal carbonate.

12. A process according to claim 11, wherein sodium methylate, sodium ethylate, potassium methylate, potassium ethylate, potassium tert. butylate, triethylamine, 1,5-diazabicyclo[4.3.0]non-5-ene, 1,8-diazabicyclo[5.4.0]undec-7-ene, sodium or potassium hydroxide, or sodium or potassium carbonate is used as the base.

13. Compounds of the general formula 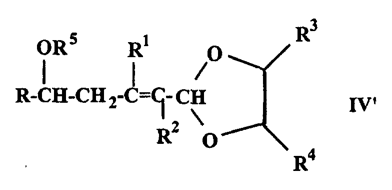 wherein
R signifies a group (a) or (b) 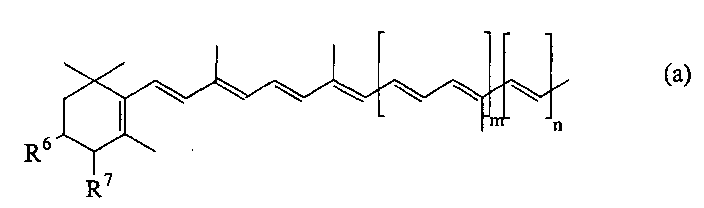 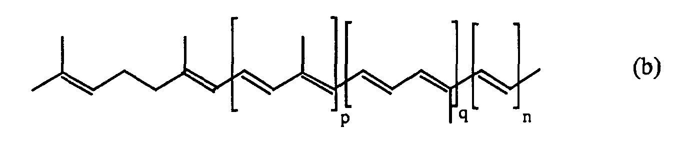 and
R¹ and R² each signify hydrogen or methyl,
R³ and R⁴ each signify hydrogen or C₁₋₄-alkyl,
R⁵ signifies C₁₋₆-alkyl,
R⁶ and R⁷ each independently signify hydrogen, an optionally protected hydroxy group or an optionally protected oxo group,
m signifies 0, 1, 2, 3 or 4,
n signifies 0 or 1,
p signifies 0, 1 or 2 and
q signifies 0, 1, 2 or 3.

14. A compound according to claim 13,
12'-methoxy-11',12'-dihydro-8'-apo-β-carotenal ethylene acetal.

## Revendications

1. Procédé de préparation d'un polyène-aldéhyde de formule générale 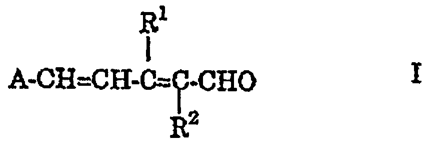 dans laquelle
A est un groupe polyène conjugué monovalent, éventuellement méthylé, et
R¹ et R² représentent chacun un atome d'hydrogène ou le groupe méthyle,
où le groupe -CH=CH-C(R¹)=C(R²)-CHO se trouve en position terminale de la chaîne conjuguée du groupe A,
**caractérisé en ce qu'**on fait réagir un polyène-O,O-éthylène-acétal de formule générale 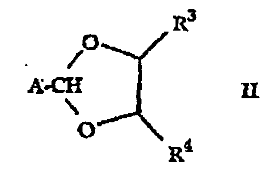 dans laquelle
A a les significations données ci-dessus, le groupe éthylène-acétal 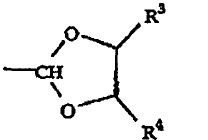 se trouvant dans ce cas sur la position terminale de la chaîne conjuguée du groupe A, et
R³ et R⁴ représentent chacun un atome d'hydrogène ou un groupe alkyle en C₁₋₄,
avec un 1-alcoxy-1,3-diène de formule générale 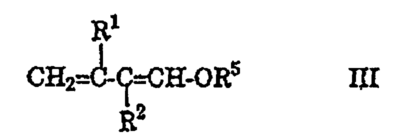 dans laquelle
R¹ et R² ont les significations données ci-dessus, et
R⁵ est un groupe alkyle en C₁₋₆,
en présence d'un acide de Brönsted, pour donner le composé de formule générale 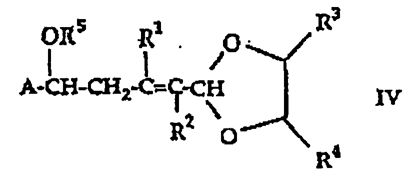 dans laquelle A, R¹, R², R³, R⁴ et R⁵ ont les significations données ci-dessus,
on hydrolyse le composé de formule IV, et, dans des conditions basiques ou acides, on élimine l'alcanol R⁵OH du composé ainsi obtenu de formule générale 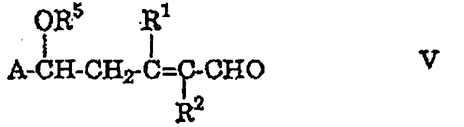 dans laquelle A, R¹, R² et R⁵ ont les significations données ci-dessus, le groupe -CH(OR⁵)-CH₂-C(R¹)=C(R²)-CHO se trouvant alors en position terminale de la chaîne conjuguée du groupe A.

2. Procédé selon la revendication 1, **caractérisé en ce que**, en tant que polyène-O,O-éthylène-acétal, on convertit un composé de formule générale 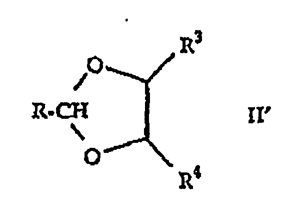 dans laquelle
R est un groupe (a) ou (b) 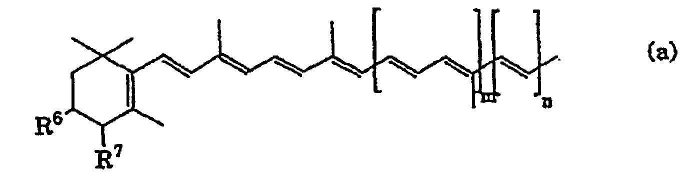 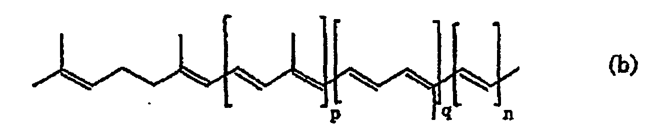 et
R³ et R⁴ ont les significations données dans la revendication 1,
R⁶ et R⁷ représentent chacun indépendamment de l'autre un atome d'hydrogène, un groupe hydroxy éventuellement protégé ou un groupe oxo éventuellement protégé,
m vaut 0, 1, 2, 3 ou 4,
n vaut 0 ou 1,
p vaut 0, 1 ou 2, et
q vaut 0, 1, 2 ou 3,
après mise en oeuvre du procédé en plusieurs étapes selon l'invention,
en le polyène-aldéhyde alicyclique-aliphatique correspondant de formule générale 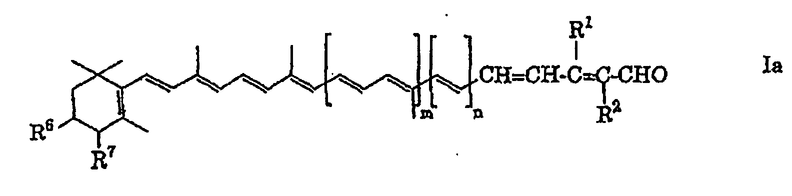 ou en le polyène-aldéhyde aliphatique correspondant de formule générale 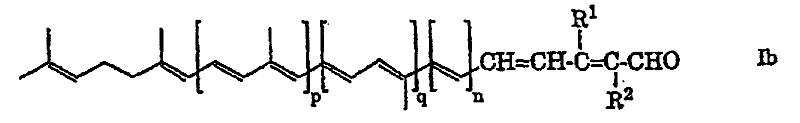 et **en ce que**, dans le cas de la présence d'un groupe (a) dans le produit de formule I, on élimine si on le souhaite un groupe protecteur éventuellement présent.

3. Procédé selon la revendication 2, **caractérisé en ce que** R est un groupe (a) dans lequel les deux radicaux R⁶ et R⁷ sont des atomes d'hydrogène, et n vaut 0.

4. Procédé selon l'une des revendications 1 à 3, **caractérisé en ce qu'**on utilise en tant qu'acide de Brönsted l'acide p-toluènesulfonique, l'acide méthanesulfonique, l'acide trifluorométhanesulfonique, l'acide sulfurique ou l'acide trifluoracétique.

5. Procédé selon l'une des revendications 1 à 4, **caractérisé en ce que** l'acide de Brönsted est utilisé en une quantité catalytique qui est comprise entre environ 0,5 et 5 % en moles par rapport à la quantité utilisée du polyène-O,O-éthylène-acétal de formule II ou II'.

6. Procédé selon l'une des revendications 1 à 5, **caractérisé en ce qu'**on fait réagir d'environ 1,05 à environ 2 équivalents de 1-alcoxy-1,3-diène par équivalent de polyène-O,O-éthylène-acétal.

7. Procédé selon l'une des revendications 1 à 6, **caractérisé en ce qu'**on fait réagir le polyène-O,O-éthylène-acétal de formule II ou II' avec le 1-alcoxy-1,3-diène de formule III dans un solvant organique à des températures comprises dans la plage d'environ -60 à environ +60°C, auquel cas on utilise en tant que solvant organique un hydrocarbure aliphatique inférieur ou cyclique, un hydrocarbure aliphatique halogéné inférieur, un éther aliphatique inférieur ou cyclique, un nitrile aliphatique inférieur ou un hydrocarbure aromatique.

8. Procédé selon la revendication 7, **caractérisé en ce qu'**on utilise en tant que solvant organique le pentane, l'hexane, le cyclohexane, le chlorure de méthylène, le chloroforme, le diéthyléther, le tert-butylméthyléther, le tétrahydrofuranne, l'acétonitrile ou le toluène, la réaction étant mise en oeuvre dans la plage de températures allant d'environ -20°C à la température ambiante.

9. Procédé selon l'une des revendications 1 à 8, **caractérisé en ce que**, immédiatement après la fin de la réaction du polyène-O,O-éthylène-acétal de formule II ou II' avec le 1-alcoxy-1,3-diène de formule III, on hydrolyse dans son propre mélange réactionnel le produit intermédiaire qui en résulte, en ajoutant au mélange réactionnel une solution aqueuse d'un acide faible, puis en agitant le mélange dans la plage de températures allant d'environ 0°C à la température ambiante.

10. Procédé selon la revendication 9, **caractérisé en ce qu'**on utilise en tant que solution aqueuse d'un acide faible une solution aqueuse faiblement diluée d'acide acétique.

11. Procédé selon l'une des revendications 1 à 10, **caractérisé en ce qu'**on met en oeuvre l'élimination de l'alcanol R⁵OH à partir du composé de formule V en faisant réagir le composé de formule V, dissous dans un solvant organique, en présence d'une quantité catalytique d'une base telle qu'un alcoolate d'un métal alcalin, une amine, un hydroxyde d'un métal alcalin ou un carbonate d'un métal alcalin.

12. Procédé selon la revendication 11, **caractérisé en ce qu'**on utilise en tant que base le méthylate de sodium, l'éthylate de sodium, le méthylate de potassium, l'éthylate de potassium, le tert-butylate de potassium, la triéthylamine, le 1,5-diazabicyclo[4.3.0]non-5-ène, le 1,8-diazabicyclo[5.4.0]undec-7-ène, l'hydroxyde de sodium ou de potassium, ou le carbonate de sodium ou de potassium.

13. Composés de formule générale 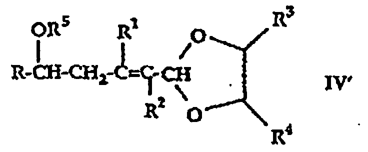 dans laquelle
R est un groupe (a) ou (b) 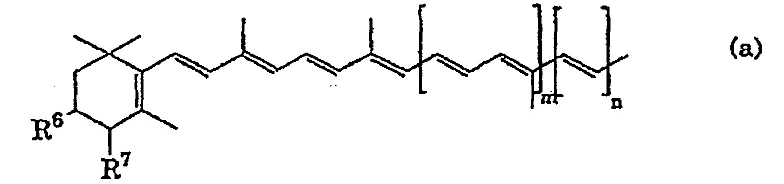 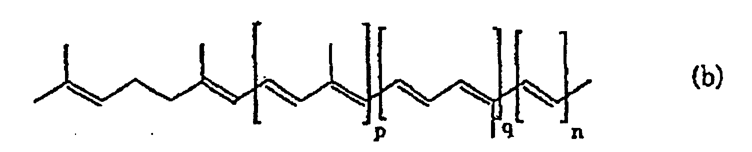 et
R¹ et R² représentent chacun un atome d'hydrogène ou le groupe méthyle,
R³ et R⁴ représentent chacun un atome d'hydrogène ou un groupe alkyle en C₁₋₄,
R⁵ est un groupe alkyle en C₁₋₆,
R⁶ et R⁷ représentent chacun indépendamment de l'autre un atome d'hydrogène, un groupe hydroxy éventuellement protégé ou un groupe oxo éventuellement protégé,
m vaut 0, 1, 2, 3 ou 4,
n vaut 0 ou 1,
p vaut 0, 1 ou 2, et
q vaut 0, 1, 2 ou 3.

14. Composé selon la revendication 13, le 12'-méthoxy-11',12'-dihydro-8'-apo-β-caroténal-éthylèneacétal.
